# EUROPEAN PATENT APPLICATION

(11) **EP 3 281 613 A2**
(43) Date of publication of application: **14.02.2018**
(21) Application number: 17186092.7
(22) Date of filing: 14.08.2017
(51) Int. Cl.: A61F 9/008, A61F 9/007

(54) **SMALL-GAUGE MICROSURGICAL INSTRUMENTS FOR USE IN OPHTHALMIC OR VITREORETINAL SURGERY**

(30) Priority: 12.08.2016 US 201615235694
(71) Applicant: Cygnus LP, Rosewell, GA 30076 (US)
(72) Inventor: MANSOUR, Fouad, Sandy Spring, GA Georgia 30328 (US)
(74) Representative: Carpmaels & Ransford LLP

(57) **Abstract**

A variable gauge microsurgical instrument for use in ophthalmic or vitreoretinal surgery is provided herein. In one aspect, a surgical probe may include a hand piece and a probe tip attached to the hand piece. A functioning member may be at least partially disposed within the probe tip. The surgical probe may further include a sleeve configured for substantially flush-fit engagement with a first size of a surgical point of entry, such as a cannula. The sleeve may be insertable over at least a portion of the probe tip. Further, the sleeve may have an outer diameter that is larger than an outer diameter of the probe tip. In some aspects, the sleeve may be configured to be deployed and retracted with respect to the hand piece.

## Description

### TECHNICAL FIELD

The present disclosure relates generally to ocular surgery devices and, more particularly, to variable-gauge microsurgical instruments for use in ophthalmic or vitreoretinal surgery.

### BACKGROUND

A common treatment often utilized in ophthalmic and vitreoretinal surgery is that of directing laser energy to a surgical site, the targeted surgical site typically being proximate a patient's retina and the surrounding vitreous. Such a surgery is called an endo-ocular photocoagulation procedure, and may be indicated for reattachment of a detached retina, for cauterization of a ruptured blood vessel, for repair of a surgical wound, for removal of defective tissue or vitreous material, and the like.

In order to conduct the endo-ocular photocoagulation procedure, or other type of ophthalmic or vitreoretinal surgery, the surgeon must utilize a microsurgical laser probe to deliver the laser energy to the surgical site within an eye. The microsurgical laser probe typically comprises a handle with a small cylindrical tip projecting from the distal end of the handle. An optical fiber element is connected at the proximal end to a laser source, and the fiber is carried through the microsurgical laser probe and into the cylindrical sleeve. The optical fiber element is positioned adjacent the distal end of the cylindrical tip in order to effectively deliver laser energy to the intended surgical site.

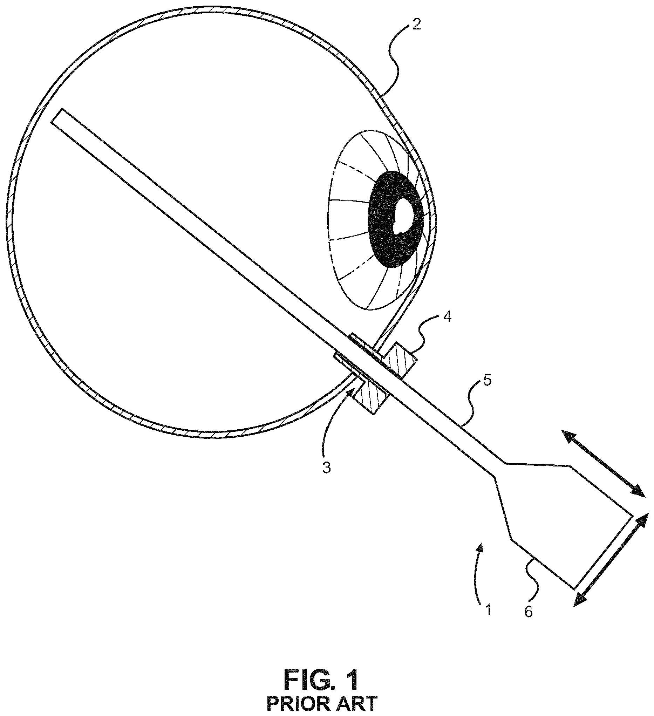

In a typical ophthalmic or vitreoretinal surgery, as shown in FIG. 1, a probe tip 5 of a surgical instrument 1 is inserted into an eye 2 via a trocar cannula 4 positioned in an entry point 3 made in the eye 2 (or in some cases via direct contact with the entry point 3). After the probe tip 5 is inserted into the eye 2, the surgeon manipulates a hand piece 6 of the surgical instrument 1 to pivot and/or rotate the probe tip 5 at its junction with the trocar cannula 4 and thus move the probe tip 5 to the desired location within the eye 2. In some cases, the probe tip 5 may be inserted into the eye 2 at variable depths , without any rotation. As an example, during a vitrectomy surgery, the surgeon manipulates the hand piece 6 to "chase" the vitreous humor with the probe tip 5. In order to consistently and effectively maneuver the probe tip 5 of the surgical instrument 1 within the eye 2, the gauge size of the probe tip 5 preferably corresponds with the inner diameter of the trocar cannula 4 (or direct entry point 3).

Ophthalmic and vitreoretinal surgery may be performed using a variety of sizes and types of probe tips. Currently, microsurgical laser probe tips are available in several predominant sizes, such as: 20 gauge (0.0360 inches), 23 gauge (0.0255 inches), 25 gauge (0.0205 inches), and 27 gauge (0.0165 inches). In some cases, even smaller gauge sizes may be used. The selection of which size and/or type of microsurgical laser probe tip to use in a surgery may be based on the nature of the procedure (e.g., the size of the trocar cannula most appropriate for a particular procedure) as well as the personal preference of the surgeon. For example, a surgeon may have been trained in and become accustomed to performing a particular procedure using a 23 gauge probe tip. Since the maneuverability and flexibility of a probe tip is affected by the gauge of the probe tip, the surgeon may experience difficultly in effectively performing the procedure with a differently sized probe tip than he or she is used to. Similarly, one type of ophthalmic or vitreoretinal surgery may favor one size of probe tip while another type may favor another size of probe tip.

In a conventional arrangement, a hospital inventory would have to maintain each type of microsurgical laser instrument with each size of probe tip. This may impose substantial burden in terms of cost, space, and inventorying effort. Thus, there is a need to provide microsurgical instruments with probe tips that can flexibly accommodate the varying preferences of multiple surgeons as well as the requirements of different types of ophthalmic or vitreoretinal surgeries.

### SUMMARY

A variable gauge microsurgical probe use in ophthalmic or vitreoretinal surgery is provided herein. In one aspect, a surgical probe may include a hand piece and a probe tip attached to the hand piece. A functioning member, such as an optic fiber, may be at least partially disposed within the probe tip. The surgical probe may further include a sleeve configured for a substantially flush-fit engagement with a first size of a surgical point of entry, such as a cannula. The sleeve may be insertable over at least a portion of the probe tip. Further, the sleeve may have an outer diameter that is larger than an outer diameter of the probe tip.

Also provided herein is a method of performing ophthalmic or vitreoretinal surgery using a surgical probe having a hand piece and a probe tip attached to the hand piece and carrying a functioning member, such as an optic fiber providing a laser or other type of light energy. The method may include inserting a sleeve over at least a portion of the probe tip. The sleeve may have an outer diameter that is larger than an outer diameter of the probe tip. The method may further include inserting the probe tip through a surgical point of entry, such as a cannula, until the sleeve realizes a substantially flush-fit engagement with the surgical point of entry. The surgical probe may be manipulated with the sleeve in flush-fit engagement with the surgical point of entry to effectuate the ophthalmic or vitreoretinal surgery.

In further aspects of the above surgical probe or method, the sleeve may be removably coupled to the probe tip of the hand piece. The sleeve may be removably coupled to the probe tip or the hand piece via a threading provided on the sleeve, a magnet provided on the sleeve or the hand piece, or a friction fit between the sleeve and the probe tip.

The sleeve may have a gauge size between 19 gauge and 34 gauge. In some aspects, the sleeve may have a gauge size smaller than 34 gauge.

The sleeve may be configured to be deployable and retractable with respect to the hand piece such that, in a first position, the sleeve is retracted at least partially within the hand piece and, in a second position, the sleeve is deployed from the hand piece and over at least a portion of the probe tip. The sleeve may be deployed and retracted with respect to the hand piece via a manipulation mechanism. The manipulation mechanism may include a sliding member associated with the hand piece and operatively coupled with the sleeve, threading associated with the sleeve, or a gear rack associated with the sleeve and a rotating element engaged with the gear rack.

In some aspects, the sleeve may comprise an outer sub-sleeve and an inner sub-sleeve movably positioned within the outer sub-sleeve. The inner sub-sleeve may cover at least a portion of the probe tip. The inner sub-sleeve may be configured for a substantially flush-fit engagement with a first size of a surgical point of entry and the outer sub-sleeve may be configured for a substantially flush fit engagement with a second size of a surgical point of entry. The sleeve comprising the inner and outer sub-sleeves may be configured such that at least one of the sub-sleeves may be deployed and retracted with respect to the hand piece. Thus, in a first position, at least one of the sub-sleeves may be retracted at least partially within the hand piece and, in a second position, the at least one of the sub-sleeves may be deployed a least partially from the hand piece and over at least a portion of the probe tip. Further, the inner sub-sleeve may be deployable and retractable with respect to the outer sub-sleeve.

The inner sub-sleeve and the outer sub-sleeve may each have a gauge size between 19 gauge and 34 gauge, wherein the gauge size of the outer sub-sleeve is larger than the gauge size of the inner sub-sleeve. In some aspects, the inner sub-sleeve and/or the outer sub-sleeve may each have a gauge size smaller than 34 gauge.

Various additional features and advantages will become more apparent to those of ordinary skill in the art upon review of the following detailed description of the illustrative embodiments taken in conjunction with the accompanying drawings.

### BRIEF DESCRIPTION OF THE DRAWINGS

The following detailed description is better understood when read in conjunction with the appended drawings. For the purposes of illustration, examples are shown in the drawings; however, the subject matter is not limited to the specific elements and instrumentalities disclosed. In the drawings:
FIG. 1 illustrates a prior art surgical instrument being used to perform an ophthalmic or vitreoretinal surgery;
FIGS. 2A, 2B, and 2C illustrate a partial cut-away view of a surgical instrument according to an embodiment of the present disclosure;
FIGS. 3A, 3B, and 3C illustrate a partial cut-away view of a surgical instrument according to an embodiment of the present disclosure;
FIGS. 4A, 4B, and 4C illustrate a partial cut-away view of a surgical instrument according to an embodiment of the present disclosure; and
FIGS. 5A, 5B, 5C, and 5D illustrate a partial cut-away view of a surgical instrument according to an embodiment of the present disclosure.

### DETAILED DESCRIPTION

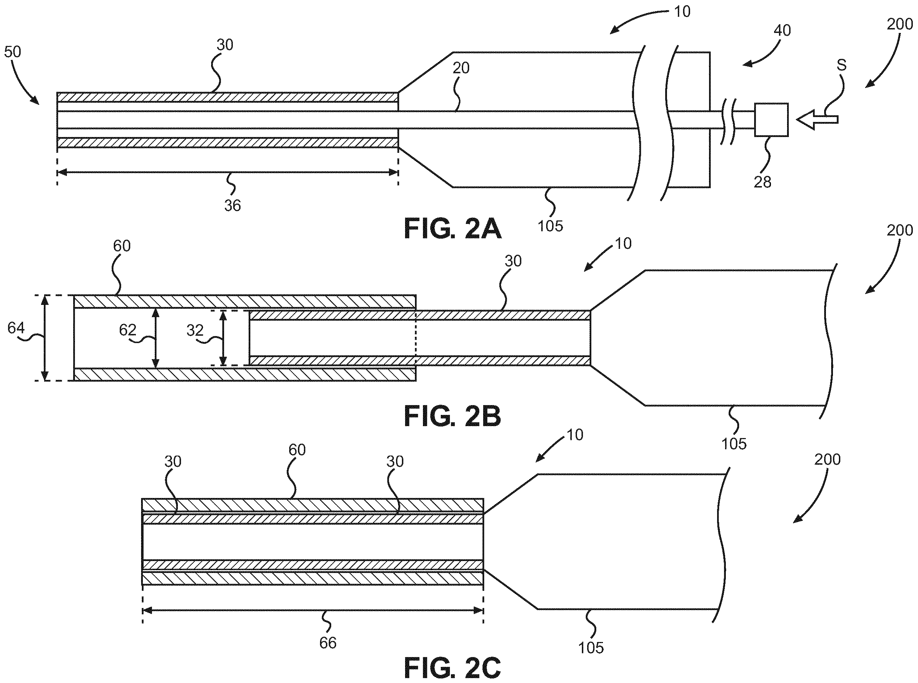

In a form of the present disclosure chosen for purposes of illustration, an exemplary embodiment 200 of which is illustrated in FIGS. 2A, 2B, and 2C, a variable-gauge microsurgical instrument for use in ophthalmic or vitreoretinal surgery is shown. A hand-held surgical instrument 10 may connect one or more of a light and a laser source S through one or more optical fibers 20 via one or more connectors 28 disposed from a proximal end 40 of the surgical instrument 10, with the distal, delivery end 50 of the surgical instrument for use inside the eye when held by a surgeon at the hand piece 105. The laser energy may, for example, be used for ophthalmic and vitreoretinal procedures involving the retina, surrounding tissue, and vitreous. Illumination energy may be supplied to illuminate the targeted surgical site. Exemplary of such a combined laser and illumination energy delivery device is Applicant's United States Patent Application Serial Number 11/934,761, filed on November 3, 2007, now United States Patent 8,647,333, the disclosure of which is hereby incorporated by reference. In other embodiments, one or more dedicated illumination optical fibers may run parallel to one or more dedicated laser energy optical fibers and connect to respective illumination and laser sources.

The surgical instrument 10 is configured with a probe tip 30 fixed to the hand piece 105 and carrying a functioning member, such as the optical fiber 20. The probe tip 30 may be formed as a tube, for example, with the optical fiber 20 or other type of functioning member being disposed therein. The optical fiber 20 may be co-terminus with the probe tip 30. The probe tip 30 may be sized according to one of an industry standard size (e.g., 19, 20, 23, 25, or 27 gauge), but is not so limited. It is further contemplated that the probe tip 30, as well as the below-described sleeve, may be sized according to future industry standard sizes as they might evolve, for example, due to a trend of miniaturization. Accordingly, the probe tip 30 or sleeve may be sized at 31, 34, or smaller gauge. The probe tip 30 may be formed with a curvature towards the distal end 50, while in other aspects, the probe tip 30 may be generally straight. Further, according to the gauge size of the probe tip 30, the probe tip 30 may exhibit varying degrees of flexibility.

While the surgical instrument 10 is generally described as encompassing a surgical instrument with a probe tip configured with an optic fiber to deliver laser or other light energy, this is just one illustrative embodiment and the disclosure is not so limited. For example, the probe tip 30 may be configured to provide one or more types of functioning members for effectuating an ophthalmic or vitreoretinal surgery, in addition or in alternative to the optic fiber 20. Examples of such a functioning member may include a vitrectomy probe, a diathermy probe, or an instrument, such as scissors or a pick.

It will be understood that references to the gauge or size of the probe tip 30 or the below-described sleeve generally describe the outer diameter of such component, unless otherwise indicated explicitly or by context. Further, however, references to the gauge or size of a cannula or other surgical point of entry through which the probe tip 30 and/or sleeve are inserted generally describe the inner diameter of such component, again unless otherwise indicated explicitly or by context. More particularly, the gauge or size of a cannula, etc. is generally described according to the gauge or size of the probe tip 30, sleeve, or other insertable component that the cannula, etc. is designed to securely but movably accommodate. Thus, for example, a 25 gauge probe tip 30 may be paired with a 25 gauge cannula such that the inner diameter of the 25 gauge cannula is about the same or slightly larger than the outer diameter of the 25 gauge probe tip 30, thus allowing the 25 gauge probe tip 30 to be freely inserted and retracted through the cannula with minimal "wiggle" or "play."

As shown in FIGS. 2B and 2C, the surgical instrument 10 may include a sleeve 60 that is positioned over the probe tip 30 of the surgical instrument 10 to increase the gauge of the probe tip 30, such as to accommodate a surgeon's personal gauge preference or to comply with the requirements of a particular type of surgery (e.g., to match the gauge size of a cannula or other surgical point of entry). In the embodiment shown in FIGS. 2B and 2C, the sleeve 60 is formed as a separate component from the probe tip 30 and the hand piece 105 and may be inserted over and/or removed from the probe tip 30 as needed. Accordingly, the sleeve 60 may be attached to the probe tip 30 and/or the hand piece 105 using various means amenable to readily attaching and detaching the sleeve 60 to the probe tip 30 and/or the hand piece 105. For example, the sleeve 60 and the hand piece 105 may each be configured with cooperatively-engaging threading to removably attach the sleeve 60 to the hand piece 105. As another example, at least one of the sleeve 60 or hand piece 105 may be configured with a magnet to removably attach the sleeve 60 to the hand piece 105. As yet another example, the sleeve 60 and the probe tip 30 may each be sized so that the sleeve 60 is sufficiently secured over the probe tip 30 via a friction fit therebetween.

In other aspects, the sleeve 60 may be coupled with the probe tip 30 and/or hand piece 105 in a more permanent manner. For instance, the sleeve 60 may be attached to the probe tip 30 and/or hand piece 105 using an adhesive.

The sleeve 60 may be sized to be securely, but movably, inserted over the probe tip 30. To this end, the inner diameter 62 of the sleeve 60 may be sized to be approximately the same as or slightly larger than the outer diameter 32 of the probe tip 30. The outer diameter 32 of the probe tip 30 may be sized according to one of the industry standard (e.g., 19, 20, 23, 25, or 27 gauge) or smaller (e.g., 31, 34 or smaller gauge) gauge sizes so that the surgical instrument 10 may be used in a surgical procedure without the sleeve 60 if the probe tip 30 is of an appropriate and/or desirable size. The outer diameter 64 of the sleeve 60 may also be sized in one of the industry standard (e.g., 19, 20, 23, 25, or 27 gauge) or smaller (e.g., 31, 34 or smaller gauge) gauge sizes that is larger than the gauge size of the probe tip 30. For example, the outer diameter 32 of the probe tip 30 may be sized in 25 gauge while the outer diameter 64 of the sleeve 60 may be sized in 23 gauge. Thus, by adding or removing the 23 gauge sleeve 60 with the 25 gauge probe tip 30, as appropriate, such an exemplary surgical instrument may be used by both surgeons that prefer a 25 gauge tip and those that prefer a 23 gauge tip.

The sleeve 60 may be configured with a longitudinal length 66 that is substantially equal to a longitudinal length 36 of the probe tip 30. Thus, the sleeve 60 is substantially coterminous with the probe tip 30 at the distal end 50 while the sleeve 60 is substantially flush and/or secured with the hand piece 105 at its other end. In another aspect, the longitudinal length 66 of the sleeve 60 may be less than the longitudinal length 36 of the probe tip 30. In such an aspect, the sleeve 60 may be substantially flush and/or secured with the hand piece 105 but not coterminous with the probe tip 30 at the distal end 50. The sleeve 60 being substantially flush and/or secured with the hand piece 105 may provide the benefit of structural support to the probe tip 30 and sleeve 60 so that the surgical instrument 10 may be reliably manipulated by the surgeon. If the sleeve 60 is not substantially flush and/or secured with the hand piece 105, undesirable bending might occur in the more flexible probe tip 30 near its junction with the hand piece 105 when the hand piece 105 is manipulated.

It will be appreciated that the surgical instrument 10 may be used with one or more of a plurality of sleeves 60, either with one sleeve 60 being inserted over the probe tip 30 at any given time or with multiple sleeves 60 of increasing size being progressively inserted over the probe tip 30 and the preceding sleeve(s) 60. In an aspect in which only one sleeve 60 of the plurality of sleeves 60 is inserted over the probe tip 30 at any given time, each sleeve 60 may be configured with the same inner diameter 62 for secure placement over the probe tip 30 but with different (e.g. progressively larger) outer diameters 64. In an aspect in which multiple sleeves 60 of the plurality of sleeves 60 are progressively inserted over the probe tip 30 and the preceding sleeve(s) 60, a first sleeve 60 may be configured to securely fit over the probe tip 30 (e.g., the inner diameter 62 of the first sleeve 60 may be the same as or slightly larger than the outer diameter 32 of the probe tip 30), a second sleeve 60 may be configured to securely fit over the first sleeve 60 (e.g., the inner diameter 62 of the second sleeve 60 may the same as or slightly larger than the outer diameter 64 of the first sleeve 60), and so forth.

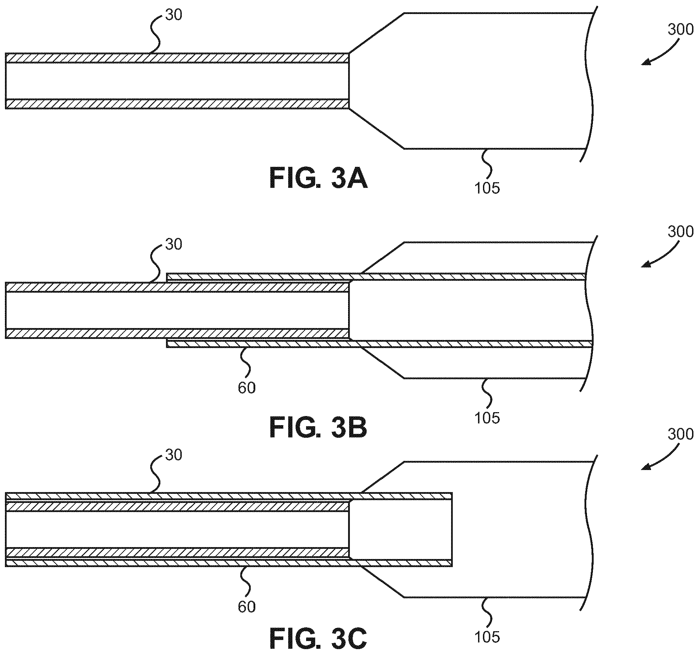

FIGS. 3A, 3B, and 3C illustrate an alternative embodiment 300 of the surgical instrument 10. Except as noted, construction of the alternative embodiment 300 is equivalent to the embodiment of FIGS. 2A, 2B, and 2C. It is noted that in the embodiment 300 shown in FIGS. 3A, 3B, and 3C, the optic fiber 20 or other functioning member has been omitted for clarity of illustration, although it is fully contemplated that the optic fiber 20 or other functioning member may be disposed within the probe tip 30. In the alternative embodiment 300, the sleeve 60 is configured to be deployed from within the hand piece 105 and retracted back into the hand piece 105, as needed. FIG. 3A depicts the sleeve 60 fully retracted in the hand piece 105. FIG. 3B depicts the sleeve 60 partially deployed over the probe tip 30. FIG. 3C depicts the sleeve 60 fully deployed from the hand piece 105 and over the probe tip 30. In some aspects, the full deployment of the sleeve 60 may bring the sleeve 60 to a point that is coterminous with the probe tip 30 at the distal end 50. In other aspects, the full deployment of the sleeve 60 may only partially cover the probe tip 30, i.e., the sleeve 60 is not coterminous with the probe tip 30 at the distal end 50. This configuration in which the sleeve 60 only partially covers the probe tip 30 may be appropriate, for example, when the portion of the probe tip 30 near the distal end 50 is curved.

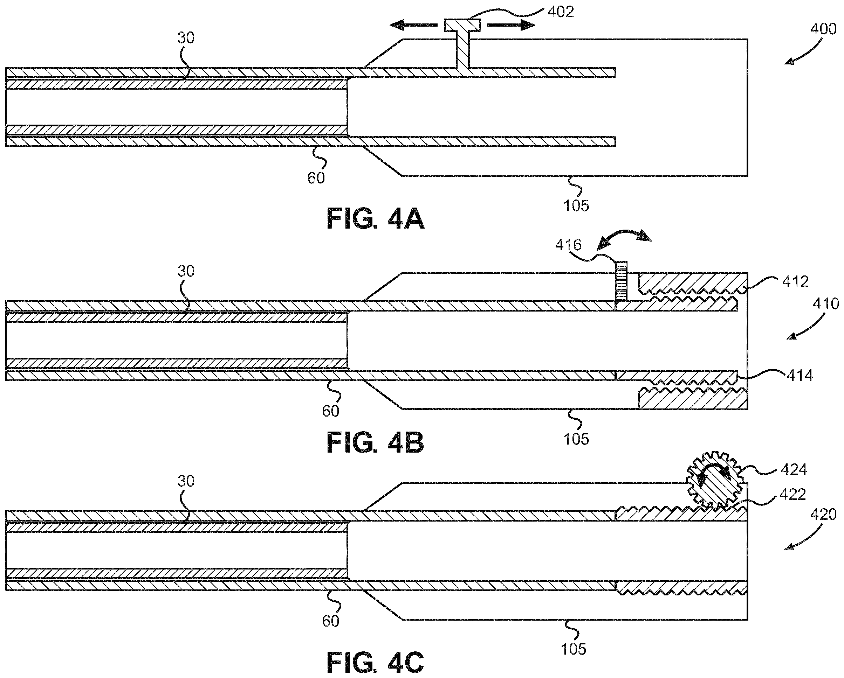

FIGS. 4A, 4B, and 4C illustrate alternative embodiments 400, 410, and 420 of the embodiment 300 shown in FIGS. 3A, 3B, and 3C. In particular, the embodiments 400, 410, and 420 illustrate various mechanisms by which the sleeve 60 may be deployed from and retracted into the hand piece 105. Except as noted, construction of the alternative embodiments 400, 410, and 420 are equivalent to the embodiment 300 of FIGS. 3A, 3B, and 3C. It is again noted that the depiction of the optic fiber 20 or other functioning member has been omitted from the embodiments 400, 410, and 420 for clarity of illustration and that it is fully contemplated that the optic fiber 20 or other functioning member may be disposed within the probe tip 30.

The embodiment 400 shown in FIG. 4A is configured with a sliding member 402 associated with the hand piece 105 and the sleeve 60 such that the sliding member 402 may be manipulated to cause the deployment and/or retraction of the sleeve 60 with respect to the hand piece 105. In particular, the sliding member 402 may be operatively coupled with the sleeve 60. Thus, when the sliding member 402 is moved back and forth with respect to the hand piece 105, the sleeve 60 is correspondingly deployed from or retracted into the hand piece 105.

In the embodiment 410 shown in FIG. 4B, a thread mechanism may be employed to effectuate the deployment or retraction of the sleeve 60. A portion of the sleeve 60 may be configured with external threads 414. The interior of the hand piece 105 may be configured with internal threads 412 that cooperatively engages with the external threads 414 of the sleeve 60. As the sleeve 60 is turned relative to the hand piece 105 (or vice versa), the engagement of the external threads 414 and internal threads 412 convert this rotational movement into linear movement of the sleeve 60 along its longitudinal axis, i.e., the deployment or retraction of the sleeve 60. The hand piece 105 may be configured with a dial 416 or other rotational element that engages with the sleeve 60 to cause rotation of the sleeve 60 when the dial 416 is rotated. At least a portion of the dial 416 or other rotational element may be positioned on the external surface of the hand piece 105 so that a user holding the hand piece 105 may rotate the dial 416 or other rotational element. Additionally or alternatively, the internal threads 412 may be rotated in-situ, thus causing the sleeve 60 to move along its longitudinal axis and deploy or retract with respect to the hand piece 105.

In the embodiment 420 shown in FIG. 4C, a gear rack mechanism may be used to deploy the sleeve 60 from the hand piece 105 onto the probe tip 30 or to retract the sleeve 60 back into the hand piece 105. In such a configuration, the sleeve 60 may be configured with a gear rack 422 and the hand piece 105 may be configured with a gear 424. The gear 424 may be cooperatively engaged with the gear rack 422 such that rotation of the gear 424 causes linear movement of the sleeve 60 along its longitudinal axis. That is, when the gear 424 is rotated, the sleeve 60 is deployed from or retracted into the hand piece 105.

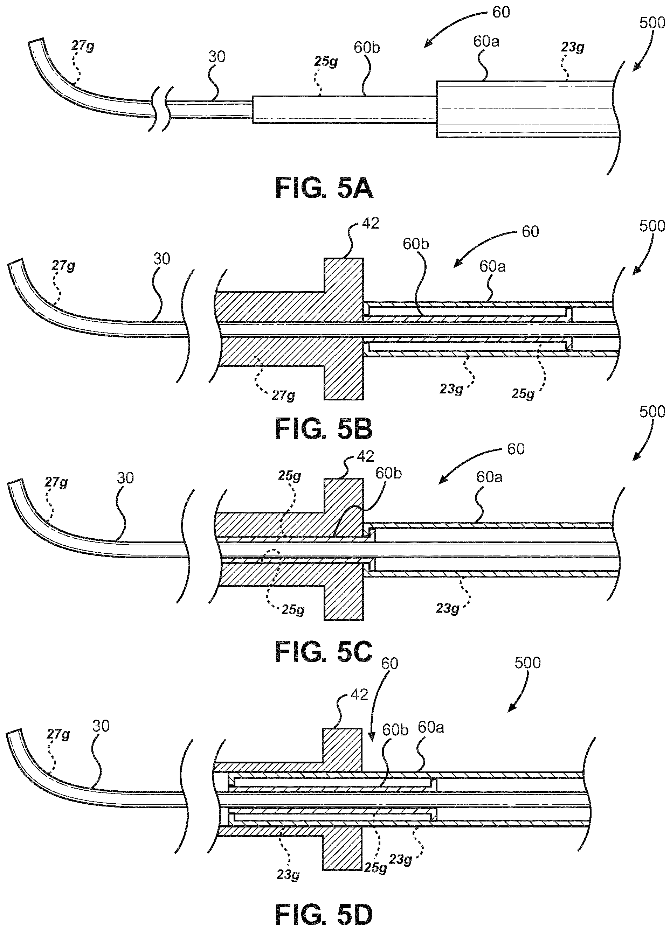

FIGS. 5A, 5B, 5C, and 5D illustrate an embodiment 500 of the surgical instrument 10 in which the sleeve 60 is configured with two or more sub-sleeves of graduated sizes. Except as noted, construction of the alternative embodiment 500 is generally equivalent to the embodiment 200 of FIGS. 2A, 2B, and 2C or the embodiment 300 of FIGS. 3A, 3B, and 3C. It is again noted that the depiction of the optic fiber 20 or other functioning member has been omitted from the embodiment 500 shown in FIGS. 5A, 5B, 5C, and 5D for clarity of illustration. Yet, it is fully contemplated that the probe tip 30 may include the optic fiber 20 or other functioning member disposed therein. It will be appreciated that while the sleeve 60 is depicted in the embodiment 500 as comprising two sub-sleeves, the disclosure is not so limited and it is explicitly contemplated that a sleeve may include three or more sub-sleeves assembled and used according to the concepts and principles described herein.

In FIG. 5A, the embodiment 500 of the instrument is configured with the sleeve 60 having an outer sub-sleeve 60a and an inner sub-sleeve 60b movably disposed inside the outer sub-sleeve 60a. The outer sub-sleeve 60a may be sized in an industry standard (e.g., 19, 20, 23, 25, or 27 gauge) or smaller (e.g., 31, 34 or smaller gauge) gauge size and the inner sub-sleeve 60b may be sized in a second, smaller such gauge size. For example and as depicted in FIGS. 5A, 5B, 5C, and 5D, the outer sub-sleeve 60a may be sized at 23 gauge and the inner sub-sleeve 60b may be sized at 25 gauge. Further in this example, the probe tip 30 may be sized at 27 gauge. Accordingly, this example configuration of the embodiment 500 may be used with a 23 gauge cannula, a 25 gauge cannula, or a 27 gauge cannula, representing a significant improvement to the usefulness of the surgical instrument 10 over a conventional surgical instrument.

As noted above, the embodiment 500 may generally derive from the embodiment 200 shown in FIGS. 2A, 2B, and 2C. Accordingly, the sleeve 60 with sub-sleeves 60a, 60b may be removably or non-removably attached to the probe tip 30 and/or the hand piece 105 in the manners described with respect to the embodiment 200.

As also noted above, the embodiment 500 may generally derive from the embodiment 300 shown in FIGS. 3A, 3B, and 3C such that the sleeve 60 with sub-sleeves 60a, 60b of the embodiment 500 may be deployable from and retractable within the hand piece 105. For example, in a first position, both the inner sub-sleeve 60b and the outer sub-sleeve 60a may be disposed within the hand piece 105 (i.e., previously retracted). This first position may be useful, for example, when the cannula used in a surgery is of a gauge size corresponding to the gauge size of the probe tip 30. In a second position, the inner sub-sleeve 60b may be deployed from the hand piece 105 and over the probe tip 30 while the outer sub-sleeve 60a remains retracted within the hand piece 105. This second position may be appropriate, for example, when a cannula used in a surgery corresponds to the gauge size of the inner sub-sleeve 60b. In a third position, the outer sub-sleeve 60a may be deployed from the hand piece 105 and over the inner sub-sleeve 60b. This third position may allow, for example, the surgical instrument 10 to be used with a cannula corresponding in gauge size to that of the outer sub-sleeve 60a. In an alternative third position, the outer sub-sleeve 60a may be deployed from the hand piece 105 but instead of the outer sub-sleeve 60a moving over and covering the inner sub-sleeve 60b, the inner sub-sleeve 60b moves correspondingly forward along the probe tip 30 such that the outer sub-sleeve 60a covers one portion of the probe tip 30 and the inner sub-sleeve 60b covers a second portion of the probe tip 30 nearer the distal end 50 of the probe tip 30.

In another example configuration of the embodiment 500, in a first position, both the inner sub-sleeve 60b and the outer sub-sleeve 60a may be disposed within the hand piece 105. In a second positon, the inner sub-sleeve 60b and the outer sub-sleeve 60a may both be deployed together from the hand piece 105 and over the probe tip 30. That is, the inner sub-sleeve 60b remains within the outer sub-sleeve 60a as the sleeve 60 is deployed to the second position from the hand piece 105. In a third position, the outer sub-sleeve 60a remains stationary in its position from the second position while the inner sub-sleeve 60b is deployed from the outer sub-sleeve 60a and further along the probe tip 30. Thus, the sleeve 60 and sub-sleeves 60a, 60b may be deployed from the hand piece 105 in a telescopic manner.

FIG. 5B illustrates one exemplary use of the embodiment 500 with a cannula 42 (or other surgical point of entry) sized at 27 gauge. In this exemplary use, the probe tip 30 is sized at 27 gauge, the inner sub-sleeve 60b is sized at 25 gauge, and the outer sub-sleeve 60a is sized at 23 gauge. Since the gauge size (27 gauge) of the cannula 42 matches that of the probe tip 30, only the probe tip 30 is inserted through the cannula 42 to realize a substantially flush-fit engagement with the cannula 42. Accordingly, the inner sub-sleeve 60b may remain disposed within the outer sub-sleeve 60a. In a configuration in which the sleeve 60 is retractable within the hand piece 105, the sleeve 60 may remain retracted within the hand piece 105 while the probe tip 30 is inserted through the cannula 42 and the surgery is performed.

FIG. 5C illustrates another exemplary use of the embodiment 500 with the cannula 42. In this exemplary use, the cannula 42 is sized at 25 gauge, the probe tip 30 is sized at 27 gauge, the inner sub-sleeve 60b is sized at 25 gauge, and the outer sub-sleeve 60a is sized at 23 gauge. Here, the gauge size (25 gauge) of the inner sub-sleeve 60b corresponds to that of the cannula 42. Therefore, the inner sub-sleeve 60b may be deployed from the outer sub-sleeve 60a and inserted through the cannula 42 to effectuate the surgery. Depending on the particular configuration of the embodiment 500, the outer sub-sleeve 60a may remain retracted within the hand piece 105 while the inner sub-sleeve 60b is deployed over the probe tip 30 or the inner sub-sleeve 60b and the outer sub-sleeve 60a may both be deployed from the hand piece 105 and over the probe tip 30 in a telescoping manner. Notably, the matching gauge size of the cannula 42 and the inner sub-sleeve 60b provides a substantially flush-fit engagement therebetween with minimal play, thus affording the surgeon optimal control of the probe tip 30 within the eye. As used herein with respect to the insertion of a component (e.g., the probe tip 30, the sleeve 60, the inner sub-sleeve 60b, or the outer sub-sleeve 60a) into the cannula 42 or other surgical point of entry, a flush-fit engagement shall be understood to mean an engagement in which the outer diameter of the component is substantially flush with the inner diameter of the cannula 42 while still allowing the component to be freely inserted into or retracted from the cannula 42.

FIG. 5D illustrates yet another exemplary use of the embodiment 500 with the cannula 42. In this exemplary use, the cannula 42 is sized at 23 gauge, the probe tip 30 is sized at 27 gauge, the inner sub-sleeve 60b is sized at 25 gauge, and the outer sub-sleeve 60a is sized at 23 gauge. Since the gauge size (23 gauge) of the outer sub-sleeve 60a matches that of the cannula 42, the outer sub-sleeve 60a may be inserted through the cannula 42, realizing a substantially flush-fit engagement of the outer sub-sleeve 60a and the cannula 42. The inner sub-sleeve 60b may remain within the outer sub-sleeve 60a, as shown in FIG. 5D, such as in a case in which the inner sub-sleeve 60b and the outer sub-sleeve 60a were both deployed together from the hand piece 105. In other configurations of the embodiment 500 (not shown in FIG. 5D), the inner sub-sleeve 60b may be further deployed in a telescoping fashion from the outer sub-sleeve 60a and over the probe tip 30 towards the distal end 50.

It will be appreciated that the foregoing description provides examples of the disclosed system and technique. However, it is contemplated that other implementations of the disclosure may differ in detail from the foregoing examples. All references to the disclosure or examples thereof are intended to reference the particular example being discussed at that point and are not intended to imply any limitation as to the scope of the disclosure more generally. All language of distinction and disparagement with respect to certain features is intended to indicate a lack of preference for those features, but not to exclude such from the scope of the disclosure entirely unless otherwise indicated.

Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context.

The following is a non-exhaustive list of embodiments which may or may not be claimed.
1. A surgical probe for use in ophthalmic or vitreoretinal surgery, the surgical probe comprising:
   a hand piece;
   a probe tip having an outer diameter and attached to the hand piece;
   a functioning member at least partially disposed within the probe tip; and
   a sleeve configured for substantially flush-fit engagement with a first size of a surgical point of entry, the sleeve being insertable over at least a portion of the probe tip, and the sleeve having an outer diameter that is larger than the outer diameter of the probe tip.
2. The surgical probe of embodiment 1, wherein the sleeve is removably coupled to the probe tip or the hand piece.
3. The surgical probe of embodiment 2, wherein the sleeve is removably coupled to the probe tip or the hand piece via at least one of: threading provided on the sleeve; a magnet provided on at least one of the sleeve or the hand piece; and a friction fit between the sleeve and the probe tip.
4. The surgical probe of embodiment 1, wherein the sleeve has a gauge size between 19 gauge and 34 gauge.
5. The surgical probe of embodiment 1, wherein the sleeve is deployable and retractable with respect to the hand piece such that, in a first position, the sleeve is retracted at least partially within the hand piece and, in a second position, the sleeve is deployed from the hand piece and over at least a portion of the probe tip.
6. The surgical probe of embodiment 5, wherein the sleeve is deployable and retractable with respect to the hand piece via a manipulation mechanism, the manipulation mechanism comprising at least one of: a sliding member associated with the hand piece and operatively coupled with the sleeve; threading associated with the sleeve; and a gear rack associated with the sleeve and a rotating element engaged with the gear rack.
7. The surgical probe of embodiment 1, wherein the sleeve comprises:
   an outer sub-sleeve configured for substantially flush-fit engagement with the first size of a surgical point of entry; and
   an inner sub-sleeve configured for substantially flush-fit engagement with a second size of a surgical point of entry, the inner sub-sleeve being movably positioned within the outer sub-sleeve, and the inner sub-sleeve being in contact with and covering at least a portion of the probe tip.
8. The surgical probe of embodiment 7, wherein at least one of the outer sub-sleeve and the inner sub-sleeve is deployable and retractable with respect to the hand piece such that, in a first position, the at least one of the outer sub-sleeve and the inner sub-sleeve is retracted at least partially within the hand piece and, in a second position, the at least one of the outer sub-sleeve and the inner sub-sleeve is deployed at least partially from the hand piece and over at least a portion of the probe tip.
9. The surgical probe of embodiment 8, wherein the inner sub-sleeve is deployable and retractable with respect to the outer sub-sleeve such that, in a third position, the inner sub-sleeve is retracted at least partially within the outer sub-sleeve and, in a fourth position, the inner sub-sleeve is deployed at least partially from the outer sub-sleeve.
10. The surgical probe of embodiment 7, wherein the inner sub-sleeve has a gauge size between 19 gauge and 34 gauge, the outer sub-sleeve has a gauge size between 19 gauge and 34 gauge, and the gauge size of the outer sub-sleeve is larger than the gauge size of the inner sub-sleeve.
11. A method of performing ophthalmic or vitreoretinal surgery using a surgical probe having a hand piece and a probe tip attached to the hand piece and carrying a functioning member, the method comprising:
   inserting a sleeve over at least a portion of the probe tip, the sleeve having an outer diameter that is larger than an outer diameter of the probe tip;
   inserting the probe tip through a surgical point of entry until the sleeve realizes a substantially flush-fit engagement with the surgical point of entry; and
   manipulating the surgical probe with the sleeve in substantially flush-fit engagement with the surgical point of entry to effectuate the ophthalmic or vitreoretinal surgery.
12. The method of embodiment 11, further comprising:
   removably coupling the sleeve to the probe tip or the hand piece.
13. The method of embodiment 12, wherein the removably coupling comprises at least one of: engaging a threading provided on the sleeve; engaging a magnet provided on at least one of the sleeve or the hand piece; and forming a friction fit between the sleeve and the probe tip.
14. The method of embodiment 11, wherein the sleeve has a gauge size between 19 gauge and 34 gauge.
15. The method of embodiment 11, wherein the sleeve is configured to be deployed and retracted with respect to the hand piece, the method further comprising:
   deploying the sleeve from at least partially within the hand piece to at least partially cover the probe tip.
16. The method of embodiment 15, wherein deploying the sleeve comprises at least one of: manipulating a sliding member associated with the hand piece and operatively coupled with the sleeve; engaging a threading associated with the sleeve; and manipulating a rotating member to engage a gear rack associated with the sleeve.
17. The method of embodiment 11, wherein the sleeve comprises an outer sub-sleeve and an inner sub-sleeve movably positioned within the outer sub-sleeve, the inner sub-sleeve covering at least a portion of the probe tip, the method further comprising:
   inserting the probe tip through the surgical point of entry until at least one of the inner sub-sleeve or the outer sub-sleeve realizes a substantially flush-fit engagement with the surgical point of entry.
18. The method of embodiment 17, wherein at least one of the outer sub-sleeve and the inner sub-sleeve is configured to be deployed and retracted with respect to the hand piece, the method further comprising:
   deploying at least one of the outer sub-sleeve and the inner sub-sleeve from at least partially within the hand piece to at least partially cover the probe tip.
19. The method of embodiment 18, wherein the inner sub-sleeve is configured to be deployed and retracted with respect to the outer sub-sleeve, the method further comprising:
   deploying the inner sub-sleeve from at least partially within the outer sub-sleeve to at least partially cover the probe tip.
20. The method of embodiment 17, wherein the inner sub-sleeve has a gauge size between 19 gauge and 34 gauge, the outer sub-sleeve has a gauge size between 19 gauge and 34 gauge, and the gauge size of the outer sub-sleeve is larger than the gauge size of the inner sub-sleeve.

## Claims

1. A surgical probe for use in ophthalmic or vitreoretinal surgery, the surgical probe comprising:
a hand piece;
a probe tip having an outer diameter and attached to the hand piece;
a functioning member at least partially disposed within the probe tip; and
a sleeve configured for substantially flush-fit engagement with a first size of a surgical point of entry, the sleeve being insertable over at least a portion of the probe tip, and the sleeve having an outer diameter that is larger than the outer diameter of the probe tip.

2. The surgical probe of claim 1, wherein the sleeve is removably coupled to the probe tip or the hand piece.

3. The surgical probe of claim 1 or claim 2, wherein the sleeve is deployable and retractable with respect to the hand piece such that, in a first position, the sleeve is retracted at least partially within the hand piece and, in a second position, the sleeve is deployed from the hand piece and over at least a portion of the probe tip.

4. The surgical probe of any preceding claim, wherein the sleeve is deployable and retractable with respect to the hand piece via a manipulation mechanism, the manipulation mechanism comprising at least one of: a sliding member associated with the hand piece and operatively coupled with the sleeve; threading associated with the sleeve; and a gear rack associated with the sleeve and a rotating element engaged with the gear rack.

5. The surgical probe of any preceding claim, wherein the sleeve comprises:
an outer sub-sleeve configured for substantially flush-fit engagement with the first size of a surgical point of entry; and
an inner sub-sleeve configured for substantially flush-fit engagement with a second size of a surgical point of entry, the inner sub-sleeve being movably positioned within the outer sub-sleeve, and the inner sub-sleeve being in contact with and covering at least a portion of the probe tip.

6. The surgical probe of claim 5, wherein at least one of the outer sub-sleeve and the inner sub-sleeve is deployable and retractable with respect to the hand piece such that, in a first position, the at least one of the outer sub-sleeve and the inner sub-sleeve is retracted at least partially within the hand piece and, in a second position, the at least one of the outer sub-sleeve and the inner sub-sleeve is deployed at least partially from the hand piece and over at least a portion of the probe tip.

7. The surgical probe of claim 6, wherein the inner sub-sleeve is deployable and retractable with respect to the outer sub-sleeve such that, in a third position, the inner sub-sleeve is retracted at least partially within the outer sub-sleeve and, in a fourth position, the inner sub-sleeve is deployed at least partially from the outer sub-sleeve.

8. A method of performing ophthalmic or vitreoretinal surgery using a surgical probe having a hand piece and a probe tip attached to the hand piece and carrying a functioning member, the method comprising:
inserting a sleeve over at least a portion of the probe tip, the sleeve having an outer diameter that is larger than an outer diameter of the probe tip;
inserting the probe tip through a surgical point of entry until the sleeve realizes a substantially flush-fit engagement with the surgical point of entry; and
manipulating the surgical probe with the sleeve in substantially flush-fit engagement with the surgical point of entry to effectuate the ophthalmic or vitreoretinal surgery.

9. The method of claim 8, further comprising:
removably coupling the sleeve to the probe tip or the hand piece.

10. The method of claim 8 or claim 9, wherein the sleeve is configured to be deployed and retracted with respect to the hand piece, the method further comprising:
deploying the sleeve from at least partially within the hand piece to at least partially cover the probe tip.

11. The method of claim 10, wherein deploying the sleeve comprises at least one of: manipulating a sliding member associated with the hand piece and operatively coupled with the sleeve; engaging a threading associated with the sleeve; and manipulating a rotating member to engage a gear rack associated with the sleeve.

12. The method of any one of claims 8 to 11, wherein the sleeve comprises an outer sub-sleeve and an inner sub-sleeve movably positioned within the outer sub-sleeve, the inner sub-sleeve covering at least a portion of the probe tip, the method further comprising:
inserting the probe tip through the surgical point of entry until at least one of the inner sub-sleeve or the outer sub-sleeve realizes a substantially flush-fit engagement with the surgical point of entry.

13. The method of claim 12, wherein at least one of the outer sub-sleeve and the inner sub-sleeve is configured to be deployed and retracted with respect to the hand piece, the method further comprising:
deploying at least one of the outer sub-sleeve and the inner sub-sleeve from at least partially within the hand piece to at least partially cover the probe tip.

14. The method of claim 12 or claim 13, wherein the inner sub-sleeve is configured to be deployed and retracted with respect to the outer sub-sleeve, the method further comprising:
deploying the inner sub-sleeve from at least partially within the outer sub-sleeve to at least partially cover the probe tip.

15. The surgical probe of any one of claims 5 to 7, or the method of any one of claims 12 to 14, wherein the inner sub-sleeve has a gauge size between 19 gauge and 34 gauge, the outer sub-sleeve has a gauge size between 19 gauge and 34 gauge, and the gauge size of the outer sub-sleeve is larger than the gauge size of the inner sub-sleeve.
